# EUROPEAN PATENT APPLICATION

(11) **EP 0 747 057 A2**
(43) Date of publication of application: **11.12.1996**
(21) Application number: 96109152.7
(22) Date of filing: 07.06.1996
(51) Int. Cl.: A61K 35/78

(54) **Use of extractive aromatic essences and trasudates-exudates of plants, particularly in the medical, zootechnical and agronomic fields**

(30) Priority: 09.06.1995 IT TO950485
(71) Applicant: Pecchiai, Maria Enrica, 20124 Milano (IT)
(72) Inventor: Pecchiai, Luciano, 20124 Milano (IT)
(74) Representative: Bosotti, Luciano

(57) **Abstract**

It is hereafter proposed the utilization of extractive aromatic essential oils or of plants transudates-exudates coming out from the vegetables' bark naturally or by insects stings or by tapping the trunk on purpose or by the effect of using a volatile fraction. The utilization suggested includes the dry vaporization in the medical, zootechnical and agronomical fields and/or the utilization of vegetable transudates-exudates in the agronomical field for example by dusting and sprinkling of hydroalcoholic solutions or by fertilizing irrigation.

## Description

### Field of the invention

The present invention refers to the extractive aromatic essential oils and to plants transudates-exudates, under which name intending the complex of sugary substances, mucilages, ceroids, ceroid-resinous substances, resins, gums, oily gum resins coming out from vegetables' bark naturally or by insects stings or by tapping the trunk on purpose.

### Description of the known technique

It is known that any part of a plant such as branches, buds, flowers, fruit and leaves are covered by a transudate that constitutes a protective mantle made of a ceroid substance, the so called bloom, particularly evident on the surface of some fruit such as apples, pears and grapes.

It is also known that in these parts of the plant, as well as in the roots and obviously in the sap are contained biologically active substances responsible for regulating the metabolic or functional activities of the plant. In some plants, these active principles are present under the form of aromatic essential oils in a particularly high quantity. These oils are usually obtained by a distillation of the essential parts in a flow of water vapour.

It is also known that the active principles contained in the sap and having a similar content to the aromatic essential oils can come out from the plant bark through a process of transudation-exudation: these are the resins, gum-resins with a solid and often glutinous aspect, oily-resins and oily-gum-resins, sometimes in a semi-liquid state in which non volatile or scantily volatile resins are melted with the essential oils.

The transudate-exudate can come out from the plant spontaneously but also after insects' stings or after having tapped the trunk in a workmanlike manner as to foster the sap's outflow.

It has been proved for a long time that essential oils, resins and gum-resins have a biological activity, so as to be employed, particularly in the past, to cure various pathologies in the human medical and veterinary fields, blending them with ointments or injectable solutions to be administered by aerosol, as inhalations.

In the agronomic field, particularly in natural, biological, biodynamic and eubiotic agricultures, the use of aromatic plants and herbs macerations but also of ethereal oils and essential oils has been suggested and carried out blending them with hydro-alcoholic solutions for sprinklings against cryptogamic and parasitic pathologies.

### Aims and summary of the invention

The present invention, having the characteristics specified in the following patent claims, basically intends to enlarge the possible fields of application of the substances previously described at least in two different directions, namely:
- utilization in the medical, zootechnical and agronomical fields of the volatile fraction of extractive aromatic essential oils and vegetable transudates-exudates, dry vaporized, in substantial absence of combustion and carbonization, and
- utilization in the agronomical field of vegetable transudates-exudates for dusting or sprinkling of hydro-alcoholic solutions or for fertilizing irrigation.

In both aspects considered above, the present invention represents a development and a generalization of the solutions described in the previous European patent applications Nos. 96105579.5 and 96105578.7, both concerning the use of incense, and both having as holder the applicant.

### Scientific bases of the invention

Without specifically referring to any theory, it is very likely to affirm that transudates-exudates are glycosides made up of a sugary component (usually glucose and galactose) with another component called aglycon that characterizes the different biological action.

The various glycosides can be employed in a different way according to their different nutritional, metabolic or defence actions; thus, they can be used for the different vegetables'states of health for a trophic-metabolic action, for water balances, sap circulation or for attacks from various pathogen agents.

Obviously, according to the need, the various glycosides can be used alone or associated with others, exactly like it happens for the different active principles in the medical field.

It is also important to specify that transudates-exudates can be integrated with the ceroid-resinous transudates accumulated by the plant around the buds and gathered by bees to be brought to their hive; then, this substance is elaborated by bees to produce the natural product called propolis (from Latin) because it is placed in front of the hive for defence.

It is obvious that, according to the first part of the invention, transudates-exudates proposed for dusting or solubilization in water or in hydroalcoholic solutions for metabolic water balance or pathologic situations follow the same indications as the use of volatile fraction released by vaporization.

Obviously, since the volatile fraction composition is different from hydroalcoholic extracts or from the raw substance used dry for dusting, what the plant foliar system absorbs from the oily-gum-resins is also different, considering the intervention of bacterial flora which is present on the plant surface.

In the various cases, it is not only suitable, but strongly advisable the associate use of the powdered product with the hydroalcoholic solution and with the volatile fraction, since this fraction can only be used in a close environment, such as greenhouses or storage rooms.

In the agronomic field, the use of aromatic essential oils and transudates-exudates is above all effective as a defence against cryptogamic and parasitic pathologies of greenhouse crops of fruit, vegetables and flowers and during storage periods of these products, be they fruit, vegetables or flowers.

From a scientific point of view, it is easy to understand that defence factors in the animal organism towards pathogen agents (bacteria, viruses and mycetes) are similar to those in the vegetable organism.

Thus, the acquired experience in the medical and veterinary fields on the effectiveness of aromatic essential oils and of the different transudates-exudates against various pathogen agents explains the reason for extending their use to the agronomic field. However, the extension to the agronomic field is also effective for modulating sap circulation, water-balances and defence mechanisms in the plant against pathogen agents, not only microorganisms or parasites, but also chemical and physic agents.

### Detailed description of the invention

As far as the first aspect considered above is concerned, it has to be underlined that both extractive aromatic essential oils and sap transudation-exudation products (ceroid substances, ceroid-resinous substances, gum resins and oily gum resins) have the peculiarity of being variably volatile. It follows that, through a suitable dry heating of the raw natural substance, a true vaporization is achieved with a consequent release of active principles, at times for sublimation.

Thus, for a first aspect, the present invention refers to an extraction technology with a physic methodology, precisely thermal, of volatile active principles contained in raw aromatic essential oils non deterpenated and of active principles contained in different transudates-exudates (ceroid substances, ceroid-resinous substances, resins, gum resins, oily gum resins), used in a raw state and vaporized, for example with an electrical heating device such as the one described in the Italian patent No. 1 240 474.

In this case, the substance to be vaporized is gathered into a capsule, that is to say a container with the shape of a glass of small dimensions. This is usually made of a metallic material, such as aluminium, having good thermal conductivity characteristics; it follows that this container can be placed inside the heating device.

While for transudates-exudates it is enough to finely pulverize the resin or the gum resin and fill the recipient with a suitable quantity, for aromatic essential oils the obstacles deriving from their rapid volatilization must be overcome before acting.

In order to avoid that the dose to be vaporized runs out in a very short time and since aromatic essential oils are liposoluble, it is better to incorporate aromatic essential oils with a lipidic vector having a high content of fatty acids and sterols.

Preferably, according to the experiments carried out by the applicant, the following substances have proved to be particularly effective for this aim:
- raw soybean lecithin, appearing at a liquid state and at room temperature, also because it contains about 30% of soybean oil;
- anhydrous lanolin, melting at about 42° C, and
- beeswax, melting at about 62-65° C.

It is also possible to use other essential oils with a correct association of corresponding lipidic vectors, such as those above mentioned; then, the procedure must be carried out with gradually growing temperatures as to obtain the slowing of different essential oils volatilization and a gradual release of volatile active principles in connection with the different melting temperatures of these three lipidic vectors.

The lipidic vector (lanolin or beeswax) can also be usefully added, in a small quantity, also to transudates-essudates.

It goes without saying that the reference to the three lipidic vectors above mentioned is a preferential example but it must not be meant as restrictive for the significance of the present invention.

The previously described volatilization technique integrates and overcomes the present techniques used in aromatherapy in the medical field such as frictions, massages, baths, footbaths, fumigations, aerosol inhalations in aqueous substrate, etc...

Obviously, it is evident that all those curative indications for the pathological situations for which the various essences and more to the point the purified essential oils have found an application within the framework of pharmacognosy (phytotherapy and aromatherapy) and which will be referred to in the following by way of example are the same as those for which resorting to the solution described herein is suggested.

From a clinical point of view, practical results prove the difference and, in general, a better effectiveness of administering volatile active principles from aromatic essential oils and oily gum resins through the olfactory ways, thus directly reaching the central nervous system, rather than through the respiratory, digesting, percutaneous, intramuscular or intravenous ways.

It is also possible to associate to those aromatic essential oils or transudates-exudates having an antiseptic and antiparasitic function, other coadjuvant substances such as propolis, i.e. the ceroid-resinous substance gathered by bees from the plants buds that is known for having similar properties for this purpose.

As far as the second aspect above mentioned is concerned, it is to be reported on the fact that, at present, herbs and aromatic essential oils are used in biological, biodynamic and eubiotic agricultures under the form of aqueous rettings and extracts incorporated in hydroalcoholic solutions with anticryptogamic and antiparasitic aims.

On the contrary, this way of using raw transudates-exudates in their natural state, for example by vegetables dusting, by sprinkling these substances melted in water or in alcohol or for fertilizing irrigation, has never been proposed or carried out.

The utilization of transudates-exudates with the specifications here described, mainly aims at keeping and restoring the trophism in the vegetable and at its defence mechanisms against environmental pathology. In fact, these transudates-exudates are considered as defence factors produced by nature itself for the vegetable world health defence and not certainly with any finalistic purpose of curing human and animal pathologies.

In addition, it should be underlined that the use of transudates-exudates in a raw state represents an advantage, because there the whole phytocomplex is present; consequently, the restrictive use of chemically extracted active principles alone, as it occurs for deterpenated and purified essential oils, has definitely been overcome.

Furthermore, it should be remarked that extractive aromatic essential oils and ethereal oils in association with propolis have been proposed and used for a long time by natural and eubiotic agricultures.

In the context of the present invention, the main object is represented by the use of vegetable transudates-exudates coming out from the plant in a raw state, thus excluding all extraction techniques.

### Examples of use of vaporization

In the following paragraphs, it will be briefly reported on some examples of vegetable transudates-exudates and non deterpenated extractive aromatic essential oils volatile fraction use in a raw state; this utilization is obtained through dry extraction with a physic method, that is to say by heating followed by the vaporization carried out in substantial absence of combustion and carbonization, for instance with a heating oven like the one described in the Italian patent No. 1 240 474.

The benefit deriving from the administration of vaporization products obtained with this method has been proved; they have to be administered in their volatile active form for applications in human and zootechnical fields by olfactory, respiratory and percutaneous-mucuous ways and in the agronomical field by the foliar system in the case of greenhouse crops and by the surface of vegetable, fruit and flowers, in the case of storage.

It has been verified that the vaporization of active principles is preferentially obtained through a heating at temperatures of 40-60-85° C. For this purpose, aromatic essential oils incorporated with the lipic vector and transudates-exudates pulverized in a raw state are placed in metallic disposable containers (such as previously described capsules) prepared with some drops of aromatic essential oils and some grams of the lipic vector, or rather some grams of transudates-exudates to be subsequently heated.

The oven used for the heating (reference is preferably made to the device described in the previous Italian patent 1 240 474, without this being restrictive for the significance of the present invention) generally has a heating power of 9 Watt on 12 Volt and it can bring a container like a capsule having a diameter of 7-8 mm. and a height of about 2 cm. up to a maximum temperature of 80-85° C in a time interval of 1 minute, 1 minute and a half.

Then, the oven heating source is deactivated for a time interval of 3-5-10 minutes, so that also the transudate-exudate and the lipidic vector masses substantially remain at the required temperature interval for 5-10 minutes. At the end of this time interval, the heating source can be reactivated after 45-60 seconds as to bring the substrate at a temperature of 50-75° C. The repetition of this cycle of deactivation/reactivation of the heating source, under the control of a thermostatic system, allows to keep the substrate within the range of temperatures between 50-60 and 85°C; in this range of temperatures the above mentioned vaporized active principle is released, excluding all phenomena of combustion/carbonization. As for aromatic essential oils having a different degree of volatilization, lower temperatures of about 40-60° C have generally been used, always with a maximum of 85°C.

### Exemplification of the use of different transudates-exudates in the agronomic field for dusting, sprinkling of hydroalcoholic solutions or for fertilizing irrigation

Both in the open field and in the greenhouse, myrrh has been successfully used for defence against cryptogams and some viruses, by virtue of its gum resins.

Against parasites (insects and microorganisms) has been usefully utilized Pinus Silvestris transudate, thanks to its olibanum-resins, having a high content of flavonoid and terpenic glycosides, similar to those of Boswellia. Cobaiba balsam has a similar indication because its oily resins are rich in sesquiterpenes, having an antiseptic and antiparasitic action. In this sense, camphor and benzoin have also been used, for this contains benzoic and cinnamic acid.

In situations characterized by water balance troubles, such as blossom-end rot or necrosis of stem, or leaves' stipe in some plants or leaf curl or peach and apricot gummosis or again withered grapes rachid disease, the following substances have been profitably used: myrrh, benzoin and Pinus Silvestris transudate, for their glycosides containing saponins of triterpenic type and glycosides having an astringent and antiseptic action.

For the opposite situation, in order to obtain a vasodilative action, besides the antiseptic and insecticide one, Galbanum transudate has been used for its gum resins, or rather camphor tree for its borneol.

### Exemplification of use of transudates-exudates and aromatic essential oils volatile fraction in the medical, zootechnical and agronomical fields

### Aromatic essential oils

In the medical field (human and veterinary) it is well known (from aromatherapy) that various pathological situations can be treated with various aromatic essential oils, which can be used separately but more frequently associated, according to the cases.

As above mentioned, the present invention allows the aromatic essential oils to reach the nervous system directly, under the form of volatile fraction, dry vaporized by a proper device.

Following this principle, some clinical situations have been verified with the treatment indication of some particularly valuable essential oils.
Mental fatigue: sage, juniper and rosemary.
Depression: sage, lavender and sandalwood.
Influenza and common colds: thyme, eucalyptus, sage, lavender, peppermint and rosemary.
Gastrointestinal diseases (diarrhoea): cypress, juniper, peppermint and sandalwood.
Cystitis: eucalyptus, juniper, Pinus Silvestris and sandalwood.
Mycotic forms (Candida albicans): thyme, cinnamon, helichrysum and lavender.
Herpetic and aphtoid forms: eucalyptus and lavender.
Water retention and cellulitis: cypress, juniper, lavender, geranium and rosemary.
Eczema and acne: cypress, juniper, lavender and sandalwood.
Rheumatoid arthritis: cypress, juniper, eucalyptus and rosemary.
Hypertension: sage, juniper, lavender and lemon balm.
Hypotension: rosemary.

In the agronomic field, the volatile fraction use can be exemplified as follows.
Regulation of water balances and sap circulation: cypress, juniper, lavender and rosemary.
Defence against bacteric pathology: eucalyptus, lavender, sage, peppermint, rosemary, juniper and sandalwood.
Defence against viral pathology: eucalyptus and lavender.
Defence against cryptogamic pathology: lavender, tea tree, thyme and helichrysum.

### Transudates - exudates

In the agronomic field, that is in greenhouses and in storage rooms, the volatile fraction use for prevention and treatment, in the different physio-pathological situations, is a repetition of what has already been specified about the use of these transudates-exudates for sprinkling.

An evident advantage is a reduction of labour, necessary for the sprinkling and the gradual spreading of the active principles, thus excluding the concentration peak that inevitably occurs with sprinkling. Furthermore, the vaporizer's action can be potentiated, with the aid of a fan and a ionizer.

Obviously, the utilization of the volatile fraction vaporizer can be associated with sprinkling and fertilizing irrigation treatments.

Finally, it is also provided the association with propolis, which, as a matter of fact, is a transudate produced by plants around the gums for their defence and gathered by bees for the defence of their hive.

In the medical field (human and veterinary), volatile fraction use of transudates-exudates can be briefly summarized for the following physiopathological situations.
"Regenerating" Action, that is to say a stimulation both of a normal cellular increase and renewal, particularly of the nervous system, above all the central nervous system: myrrh's gum resins and Pinus Silvestris and Boswellia's olibanum resins.
"Astringent" Action, against cater retention, cutaneous streaks and cellulitis: cypress, juniper and benzoin's resins, myrrh's gum resins and Pinus Silvestris and Boswellia's olibanum resins.
Antiseptic, bacteriostatic and antiparasitic action: Galbanum's gum resins, Pinus Silvestris and Boswellia's olibanum resins, Copaiba balsam's oily resins and camphor's borneol.
Antiviral action: myrrh's gum resins.
Fungicide action (Candida albicans): myrrh's gum resins.

In the medical field too, exactly like in the agronomic field, it is provided a useful association of transudates-exudates with that transudate known as propolis which is produced by plants around the buds and then gathered and elaborated by bees.

Thus, the principle of the invention being understood, the realization details and methods to carry it out could be widely modified with respect to what has been described and illustrated, though within the field of the present invention.

## Claims

1. A process for producing an active form of an extractive aromatic essential oil and/or plants transudate-exudate (as previously defined) characterised by the step of heating said aromatic essential oil and/or said transudate-exudate causing vaporization in substantial absence of combustion and carbonization.

2. The process according to claim 1, characterized by the step in which said aromatic essential oil and/or transudate-exudate is heated at a temperature approximately between 40 and 85° C.

3. The process according to claim 1 or claim 2, characterized by the step of heating said aromatic essential oil and/or said transudate-exudate with alternating cycles of heat application phases with cooling phases.

4. The process according to claim 3, characterized by the length of said heat application phases which should be of 45-90 seconds and are separated by cooling phases lasting approximately 3-10 minutes.

5. The process according to any of the preceding claims, characterised by the use of the said aromatic essential oil and/or said transudate-exudate in a raw state, non deterpenated.

6. The process according to any of the preceding claims, characterised by the fact that, before the said vaporization, the aromatic essential oil and/or transudate-exudate are incorporated to a lipidic vector.

7. The process according to claim 6, characterised by the fact that the said lipidic vector is chosen among the following group: raw soybean lecithin, anhydrous lanolin and beeswax.

8. The process according to claim 6 or claim 7, for which at least two different aromatic essential oils and/or transudates-exudates are vaporized and characterised by the utilization of two respective lipidic vectors having different melting points.

9. The process according to any of the preceding claims, characterised by the use of said aromatic essential oil and/or transudate-exudate in association with propolis.

10. The process according to any of the preceding claims, characterised by the fact that the said aromatic essential oil is, for example, chosen among terpenic essential oils such as pine, juniper, eucalyptus, sage, lavender, peppermint, rosemary, among oxidated terpenic essential oils such as camphor or among phenolic essential oils such as cinnamon, said choice being preferably specific, as for instance: thyme, eucalyptus, lavender, peppermint and sandalwood for an antibacteric action; thyme, cinnamon, and lavender for an antimycotic action; eucalyptus and lavender for an antiviral action, said transudate-exudate being, for example, chosen among resins such as cypress, juniper and benzoin; among gum resins such as myrrh and Galbanum; among olibanum resins such as Pinus Silvestris and Boswellia; among oily gum resins such as Copaiba balsam, said choice being preferentially made aiming at reaching a general trophic action, at modulating blood circulation and sap circulation, for instance thanks to a peculiar antibacteric and antiparasitic action for Pinus Silvestris, Boswellia, Galbanum, benzoin and Copaiba balsam and thanks to a peculiar antiviral and fungicide action for myrrh.

11. Use of a vegetable transudate-exudate (as previously defined) particularly but not exclusively for vegetables' metabolic trophism and as a defence against cryptogamic and parasitic pathologies.

12. Use, according to claim 11, of said transudate-exudate in a raw state.

13. Use, according to claim 11 or claim 12, of said transudate-exudate under the form of powder or oil, or under the form of alcoholic or aqueous extract or in another solvent or as vapour.

14. Use, according to any of claims 11 to 13, of said transudate-exudate associated with propolis.

15. Use, according to claim 11, for defence against cryptogams, in which said transudate-exudate is, for example myrrh.

16. Use, according to claim 11, of said transudate-exudate for instance derived from Pinus Silvestris, Cobaiba balsam, benzoin or camphor for the defence against parasites such as microorganisms and insects.

17. Use, according to claim 11, of said transudate-exudate chosen, for example, from the group composed by myrrh, benzoin and Pinus Silvestris transudate, against water balance troubles such as blossom-end rot or necrosis of stem or leaves' stipe, leaf curl or peach and apricot gummosis or withered grapes rachid disease.

18. Use, according to claim 11, of said transudate-exudate, for example, Galbanum or camphor transudate for an antiseptic and insecticide action.

19. A process for vegetable products treatment, such as fruit and vegetables or flowers, characterised by the application of an effective quantity of vegetable transudate-exudate (as previously defined).

20. The process according to claim 19, characterised by the application to vegetable products of said transudate-exudate under the form of powder, oil or under the form of alcoholic or aqueous extract or in another solvent or as vapour.

21. The process according to claim 19, characterised by the application of said transudate-exudate by sprinkling or by fertilizing irrigation.

22. The process according to any of claims 19 to 21, characterised by the application of said transudate-exudate in association with an effective quantity of propolis.
